# EUROPEAN PATENT SPECIFICATION

(11) **EP 3 227 260 B1**
(45) Date of publication and mention of the grant of the patent: **24.02.2021**
(21) Application number: 15866045.6
(22) Date of filing: 10.11.2015
(51) Int. Cl.: C07C 67/00, C07C 59/185, C07C 51/09

(54) **PROCESS FOR THE PREPARATION OF LEVULINIC ACID**
VERFAHREN ZUR HERSTELLUNG VON LEVULINSÄURE
PROCÉDÉ POUR LA PRÉPARATION D'ACIDE LÉVULINIQUE

(30) Priority: 03.12.2014 IN 3864MU2014
(43) Date of publication of application: 11.10.2017
(73) Proprietor: Praj Industries Limited, Hinjewadi, Pune 411057 (IN)
(72) Inventor: JAYANT, Shridhar Sawant, Pune 411057 (IN); NITIN, Sudhakar Patil, Pune 411057 (IN)
(74) Representative: Dr. Klemens Schubert Patentanwalt
(86) International application number: PCT/IN2015/000415
(87) International publication number: WO 2016/088133

(56) References cited:
- WO-A1-2013/078391
- WO-A1-2013/078391
- WO-A2-2014/033734
- CN-A- 101 781 210
- US-A- 3 267 136

## Description

### FIELD OF INVENTION

The invention relates to a process for the preparation of levulinic acid and more particularly to a selective conversion of fructose to levulinic acid in a mixture of glucose and fructose.

### BACKGROUND

Levulinic acid, a keto-acid is prepared from renewable biomass. It is used to prepare esters of LA generally used as octane booster for gasoline and as a fuel extender for diesel. It is also a substrate for a variety of condensation and addition reactions involving the ester and keto groups with alkyl amines to form various polymers which have applications in various industrial processes like coatings, plastic, films, etc as well as a reactant in many chemical preparation processes to create various chemical substances that are used in consumer or pharmaceutical products.

Conversion of glucose and other hexose sugars to levulinic acid by treating hexose sugar containing streams with mineral acids appeared in the patent literature as early as 1960 (Canadian patent no. CA594974A). This method was adapted later to the direct transformation of biomass using mineral acids at high temperatures. Besides, PCT application WO2005070867 discloses reactive extraction of levulinic acid by means of esterification of C5 to C12 un-branched aliphatic alcohols with yields of levulinate esters as high as 85%. However, most of these methods require the use of corrosive acids and hazardous chemicals during the conversion of sugars to levulinates.

The international application WO 2013/078391A1 discloses a process to prepare levulinic acid, formic acid and/or hydroxymethyl furfural from various biomass materials. Said process comprises the steps of heating a mixture of mineral acid and a solution comprising water and fructose to form a reaction mixture including levulinic acid and solids, filtering the solids from the mixture, extracting the aqueous solution with an organic solvent to form first and second layer, recovering levulinic acid from the second layer, and recycling the first layer back to the reactor.

The international application WO 2014/033734A2 discloses a process and system for the preparation of esters of levulinic acid. Said process for converting sugars to esters of levulinic acid comprises mixing alcohol and glycerol with a feed stock comprising one or more sugars to create a reaction mixture of alcohol, glycerol and the feedstock is disclosed. The reaction mixture is contacted with an acid catalyst. The contact between the acid catalyst and the reaction mixture is maintained at an effective temperature and pressure for a specific time period so as to cause a desired reaction to result in a product stream. The product stream is separated into components by virtue of the difference in their boiling points, miscibility or specific gravity. Esters of levulinic acid, glycerol, alcohol and additional by-products are recovered from the product stream.

The major issues with conversion of sugars to levulinic acid relate to the selectivity of the conversion, the yields obtained, and the associated high cost of production of levulinic acid. Thus, there is still a need for a process to produce levulinic acid that is economically viable, energy efficient, environment friendly and yet capable of producing the desired product with enhanced purity and significantly increased product yields.

### BRIEF DESCRIPTION OF THE INVENTION

The present invention is defined by the claims and provides a process for converting sugars to levulinic acid. It specifically provides for selective conversion of fructose to levulinic acid in a mixture of hexose sugars like a mixture of glucose and fructose.

The present invention provides a process for the preparation of levulinic acid comprising: providing a feedstock comprising fructose and glucose; mixing methanol and an acid with said feedstock and allowing insoluble matter to separate forming a first stream; subjecting said first stream to solid-liquid separation forming a reaction mixture free of said insoluble matter; maintaining said reaction mixture at an effective temperature for a specific time period forming in a second stream, wherein the temperature is between 90°C and 120°C; subjecting said second stream to pH correction using an alkali forming a third stream; distilling said third stream forming a fourth stream and a forming a first distillate stream; filtering said fourth stream forming a fifth stream; extracting said fourth stream with an organic solvent to get a mixture of aqueous and organic phases; separating said mixture by liquid-liquid separation forming an organic stream and an aqueous stream; subjecting said organic stream to distillation to remove said organic solvent and get a product stream comprising methyl levulinate; recovering levulinic acid from said methyl levulinate by hydrolysis and removal of methanol; and recovering glucose from said aqueous stream by hydrolysis and removal of methanol.

### DESCRIPTION OF DRAWINGS

These and other features, aspects, and advantages of the present invention will become better understood when the following detailed description is read with reference to the accompanying drawings, wherein:

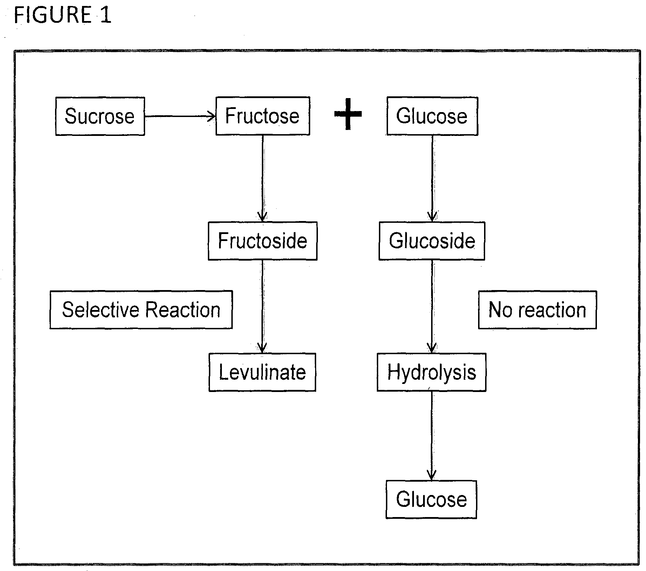

FIGURE 1 depicts a general scheme of the process of invention in that a feedstock comprising fructose and other sugars like glucose is subjected to the innovative and specific reaction conditions in that firstly fructose and glucose are covered to their respective methyl glycosides. Then in the second step, only methyl fructoside is converted selectively to methyl levulinate and methyl glucoside remains as it is which is further used to recover glucose on hydrolysis. Herein said methyl levulinate may be hydrolysed to obtain levulinic acid.

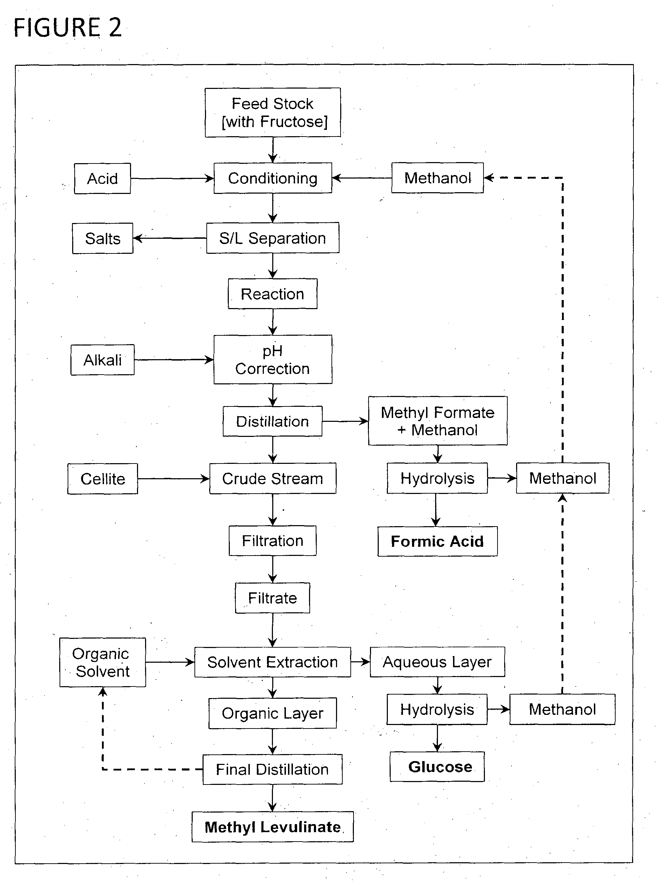

FIGURE 2 depicts a process flow diagram of the production of levulinic acid from a feedstock comprising a mixture of fructose and glucose such as molasses or invert syrup. Different elements of the process are identified and directional movement of different streams and components formed during the process are shown to describe the features of one embodiment of the present invention.

### DETAILED DESCRIPTION OF THE INVENTION

Feedstock comprising fructose may be converted in accordance with one aspect of the present invention to form levulinic acid useful for applications such as polymers, bulk chemicals, solvents, fuel additives, detergents, antifreeze agents, pharmaceutical ingredients, herbicides, plasticizers, lubricants, cleaning chemicals or resins. Examples of fructose containing feed stocks include sugarcane or sugar beet or sweet sorghum juice or syrup or molasses, dates, honey, corn, potato, cassava, sago, rice, wheat, barley, sorghum or millet wherein if only glucose is present it is inverted to fructose by enzymes.

In one embodiment of the present invention, a process for selectively converting fructose to levulinic acid comprises mixing methanol with a feed stock comprising fructose and glucose creating a reaction mixture. The mass percentage of total fructose in the reaction mixture is in a range from about 5 percent to about 25 percent. The mass percentage of water in the reaction mixture may be in a range from about 2.5 percent to about 10 percent.

In accordance with an embodiment of the present invention, the reaction mixture is contacted with an acid catalyst like sulphuric acid, hydrochloric acid or an organic acid like methane sulphonic acid. A contact between the reaction mixture and the acid catalyst is maintained at an effective temperature and pressure to cause a desired reaction forming a product stream.

According to the present invention, the contact between the reaction mixture and the acid catalyst is maintained at a temperature in the range between 90 °C and 120 °C. In another embodiment of the present invention, the contact between the reaction mixture and the acid catalyst is maintained for a time-period ranging between 20 minutes and 300 minutes.

In one embodiment of the present invention, the reaction results in a product stream comprising methyl levulinate, and other by-products. The said methyl levulinate may be hydrolysed in the presence of an acid to get levulinic acid as a final product.

In one embodiment of the present invention, alkali is added to the liquid stream to adjust pH in a range from about 6 to about 8. The alkali used may include, but is not limited to aqueous solution of ammonia, metal and alkaline metal hydroxides, such as hydroxides of sodium, potassium, calcium, lithium, and their carbonates and bicarbonates.

In a process according to one embodiment of the present invention, methyl levulinate is selectively prepared from fructose as detailed below in a system of modules, wherein each module has a specific function leading to conversion of said sugar to the methyl levulinate with high specificity and selectivity. In another embodiment of the present invention, the system may be used to recover and recycle of the methanol used in the process.

In one embodiment of the present invention, efficient conversion of fructose to methyl levulinate is obtained using a system comprising three modules namely: 1] feed preparation module; 2] reaction module; and 3] downstream processing module. Each module has one or more elements/ means for performing specific or optional functions as required for achieving the selective production of methyl levulinate at higher amounts.

A person skilled in the art may appreciate different variations and/ or combinations of these elements that may be used to perform the objects of the invention disclosed herein.

### FEED PREPARATION MODULE

Feed preparation module is used for mixing a feedstock comprising fructose and glucose with methanol and adjusting moisture content to a desired level if necessary. This is followed by conditioning leading to the formation of a reaction mixture of feedstock and methanol in a desired ratio. After a period of about 30 min to 5 h of conditioning, solid insoluble matter such as salts, settle out of said reaction mixture. These salts are removed by filtration to obtain a clarified reaction mixture. Further, it is sent to the next module called the reaction module.

In one embodiment of the present invention, a feedstock comprising liquid glucose obtained from corn starch is inverted to with enzymes to contain about 50 % fructose by volume and then mixed with in the feed preparation module leading to the creation of a reaction mixture for the preparation of methyl levulinate at higher amounts and glucose obtained as the by-products is recycled to produce fructose upon inversion, or converted ethanol by fermentation.

### REACTION MODULE

The second module of the system is a reaction module characterized by one or more high temperature and pressure vessels, capable of holding the reaction mixture, acid catalyst and a product stream and allowing a desired reaction to occur at a condition of high pressures up to 40 bars and high temperatures up to 200 °C. These vessels may be one or more of stirred tanks, plug-flow reactors, accelerated plug-flow reactors, recirculation reactors or fluidized-bed reactors. These vessels may operate in batch mode, fed-batch mode or continuous mode in parallel or in series or a combination thereof. The reaction module has means for controlling temperature and pressure at a desired level during the reaction for extended time-periods. In another embodiment of the present invention, the reaction mixture and the acid catalyst are fed into the reaction module and contact between the catalyst and the reaction mixture is maintained at an effective temperature and pressure for a specific retention time-period in the vessel to achieve a desired reaction resulting in formation a product stream, which is fed to the next module called downstream processing module.

In one embodiment of the present invention, the vessel is an autoclave vessel wherein the reaction mixture is subjected to an elevated temperature and pressure for a desired time-period in the presence of a acid catalyst held in place in the autoclave such that it optimally contacts the reaction mixtures leading to efficient production of desired products.

In another embodiment of the present invention, the vessel is a batch-type stirred tank reactor wherein a mixture of fructose and glucose and methanol are subjected to an elevated temperature and pressure for a desired time period in the presence of a acid catalyst such that it optimally contacts the reaction mixture leading to efficient production of methyl levulinate and methyl glucoside. Levulinic acid and glucose is prepared on hydrolysis of respectively products.

In yet another embodiment of the present invention, the vessel is a plug-flow reactor wherein a mixture of fructose and methanol is subjected to an elevated temperature and pressure for a desired time period in the presence of a acid catalyst such that it optimally contacts the reaction mixture leading to efficient production of methyl levulinate.

In yet another embodiment of the present invention, the vessel is a recirculation reactor wherein a mixture of liquid glucose and methanol and is subjected to an elevated temperature and pressure for a desired time period in the presence of a acid catalyst such that it optimally contacts the reaction mixture leading to efficient production of methyl levulinate.

### DOWNSTREAM PROCESSING MODULE

The downstream processing module comprises of various unit processes to enable efficient separation of the product and by-product streams. Typical unit processes include solvent extraction, filtration, neutralization and distillation units.

In one embodiment of the downstream module, the reaction mixture emerging out of the reaction module is pH corrected to a value between 6 and 8 and then subjected to distillation to remove a stream [first distillate stream] comprising methyl formate from which formic acid and methanol is recovered on acid hydrolysis. After the distillation the remaining crude stream is subjected to filtration and the filtrate is subjected to solvent extraction with an organic solvent immiscible in water. Typical solvents employed are but not limited to alkyl ketones, halogenated solvents or higher alcohols having affinity for methyl levulinate. The organic stream resulting from solvent extraction is subjected to final distillation to obtain the final product. The aqueous stream is subjected to an acid to separate glucose from methyl glucoside, which is fermented to ethanol or isomerised to fructose and recycled back to prepare levulinic acid as disclosed herein.

The methyl levulinate or levulinic acid content at the various stages in the process is analyzed in test samples by known methods. Methyl levulinate or levulinic acid is analyzed by gas chromatography with flame ionization detector. Alltech ATTM-wax column of size 60 m X 0.53mm ID and film thickness of 1 µm or any equivalent column is used. Helium is used as carrier gas with flow rate of 3 mL/min. Injector and detector temperatures are kept at 240°C and 260°C respectively. Temperature gradient program is used with total run time of about 24 minute. Retention time of methyl levulinate is observed at about 10.63 minute. Sample injection volume is about 1 µL. Dimethyl formamide is used as a diluent for the sample preparation. Estimation of methyl levulinate content in the test samples is done using calibration graph generated using at least five known concentrations of standard compound.

Glucose content is analyzed by liquid chromatography with refractive index detector. BioRad Aminex 87 H⁺ column of size 300 X 7.8 mm or any equivalent column is used with 0.005 M H₂SO₄ as mobile phase with flow rate of 0.6 mL/min. Column oven temperature is kept at 55 °C and injection volume at 20 µL. Deionised water is used as a diluent for the sample preparation. Retention time of glucose is observed at 8.9 minute. Estimation of glucose content in the test samples is done using calibration graph generated using five known concentration of standard compound

### EXAMPLES

Examples provided below give wider utility of the invention without any limitations as to the variations that may be appreciated by a person skilled in the art. A non-limiting summary of various experimental results is given in the examples, which demonstrate the advantageous and novel aspects of the process of using methanol in the selective conversion of fructose to methyl levulinate of levulinic acid.

### EXAMPLE 1 - Reference

About 1000 g of molasses containing about 487 g of total sugar was mixed with about 500 g of methanol. The reaction mixture was allowed to stand for about 5 h for the insoluble matter to settle and resulting in a supernatant liquid stream containing about 390 g of sugar (about 378 g of sucrose, 59 g fructose and about 32 g of glucose) and having ash value below about 5 % by weight. Said supernatant liquid stream was cleared by filtration. The stream so obtained was transferred to a reactor wherein about 48 g of H₂SO₄ and about 4117 g of fresh methanol was added. Next, the reactor was heated under stirred condition and maintained at about 105 °C for about 12 h. After 12 h the reaction mass was flashed or distilled to separate methyl formate and methanol from it. Remaining reaction mass, without methyl formate and methanol, was treated with about 12 g NaOH dissolved in about 2220 g water before subjecting it to complete methanol removal by distillation. Then it was extracted with ethylene dichloride or methyl isobutyl ketone to separate methyl levulinate in the organic stream leaving behind methyl glucoside in the aqueous stream. The organic stream was subjected to final distillation to separate said organic solvent from methyl levulinate. Methyl levulinate was hydrolyzed to recover about 83 g of pure levulinic acid and methanol, whereas methyl glucoside remaining in the aqueous stream was hydrolyzed using acid to recover about 159 g of D-glucose. Additional about 93 g of sugars are obtained by extracting the insoluble matter separated in the first step of methanol wash of the molasses feedstock. Thus, about 252 g of total sugar was recovered. This sugar on fermentation using yeast yielded about 117 g of ethanol. This process afforded about 90 % efficiency of methyl levulinate production from fructose with recovery of about 90 % of unreacted sugars in the form of fermentable glucose. The methanol recovered at each of the above steps was recycled.

### EXAMPLE 2 - Reference

About 1000 g of low ash sugar stream, obtained as described in EXAMPLE 1, containing about 286 g of sugar was mixed with about 3450 g of methanol and about 75 g sulphuric acid. The reaction mixture was transferred to a reactor. Next, the reactor was heated under stirred condition and maintained at about 105 °C for about 8 h. Then the reaction mass was analyzed by gas chromatography for methyl levulinate formation. The analysis of reaction mass contained about 1.4 % methyl levulinate, which corresponded to about 31 % yield of theoretical value based on the input sugars.

### EXAMPLE 3 - Reference

About 529 g of low ash sugar stream, obtained as described in EXAMPLE 1, containing about 308 g of sugar was mixed with about 3270 g of methanol and about 40 g sulphuric acid. The reaction mixture was transferred to a reactor. Next, the reactor was heated under stirred condition and maintained at about 105 °C for about 4 h. Then the reaction mass was analyzed by gas chromatography for methyl levulinate formation. The analysed reaction mass contained about 1.65 % w/w methyl levulinate, which corresponded to a yield of about 27 % of theoretical value on the input sugars. The reaction was further continued for a period of 12 h. Analysis of reaction mass after 12 h showed that it contained about 2.2 % w/w of methyl levulinate, which corresponded to about 36 % yield of theoretical value based on input sugars.

### EXAMPLE 4 - Reference

About 60 g of low ash sugar stream, obtained as described in EXAMPLE 1, containing about 15 g of sugar was mixed with about 352 g of methanol and about 8 g sulphuric acid. The reaction mixture was transferred to a reactor. Next, the reactor was heated under stirred condition and maintained at about 95 °C for about 5 h. Then reaction mass was analyzed by gas chromatography for methyl levulinate formation. The analysed reaction mass contained about 0.5 % methyl levulinate by weight, which corresponded to a yield of about 10 % of theoretical value on the input sugars. The reaction was further continued for a period of 23 h. Analysed reaction mass showed that it contained about 1 % w/w of methyl levulinate, which corresponded to about 18 % yield of theoretical value based on input sugars.

### EXAMPLE 5 - Reference

About 33 g of clarified cane syrup containing about 25 g of sugar was mixed with about 407 g of methanol and 4 g sulphuric acid. The reaction mixture was transferred to a reactor. Next, the reactor was heated under stirred condition and maintained at about 105 °C for about 4 h. After 4 h the reaction mass was analyzed using gas chromatography for methyl levulinate formation. The analysis of reaction mass contained about 1.36 % w/w methyl levulinate, which corresponded to a yield of about 33 % of theoretical value on the input sugars. The reaction was further continued for a period of 12 h. Analysed reaction mass showed that it contained about 7 % w/w of methyl levulinate, which corresponded to about 39 % yield of theoretical value based on input sugars.

### EXAMPLE 6 - Reference

About 51 g of molasses containing about 26 g of sugar was mixed with about 260 g of methanol and about 6.4 g of sulphuric acid. The reaction mixture was transferred to a reactor. Next, the reactor was heated under stirred condition and maintained at about 95 °C for about 8 h. After 8 h the reaction mass was analyzed by gas chromatography for methyl levulinate formation. The analysed reaction mass contained about 2.5 % methyl levulinate, which corresponded to about 13 % yield of theoretical value based on the input sugars.

### EXAMPLE 7 - Reference

About 1000 g of dextrose powder containing about 95 g of moisture was mixed with about 6200 g of methanol and about 36 g of methane sulphonic acid. The reaction mixture was transferred to a reactor. Next, the reactor was heated under stirred condition and maintained at about 95 °C for about 5 h. After 5 h the reaction mass was analyzed by gas chromatography for methyl levulinate formation. The analysed reaction mass contained no methyl levulinate.

### EXAMPLE 8 - Reference

About 500 g of fructose powder was mixed with about 3400 g of methanol and about 20 g of methane sulphonic acid. The reaction mixture was transferred to a reactor. Next, the reactor was heated under stirred condition and maintained at about 95 °C for about 5 h. After 5 h the reaction mass was analyzed using gas chromatography for methyl levulinate formation. The analysed reaction mass contained about 2.6 % methyl levulinate, which corresponded to about 28 % yield of theoretical value based on the input sugars.

### EXAMPLE 9 - Reference

To about 400 g of the reaction product obtained from EXAMPLE 6, about 2.5 g of methane sulphonic acid was added and the resulting mixture was heated in a stirred autoclave reactor at about 105 °C for 7 h. After 7 h the reaction mass was analyzed by gas chromatography for methyl levulinate formation. The analysed reaction mass contained about 7 % methyl levulinate, which corresponded to a yield of about 71 % of theoretical value based on the input sugars.

### EXAMPLE 10 - Reference

To about 400 g of the reaction product obtained from EXAMPLE 6 about 2.5 g of methane sulphonic acid was added and the resulting mixture was heated in a stirred autoclave reactor at 95 °C for 7 h. After 7 h the reaction mass was analyzed by gas chromatography for methyl levulinate formation. The analysed reaction mass contained about 3 % methyl levulinate, which corresponded to about 65 % yield of theoretical value based on the input sugars.

### EXAMPLE 11 - Reference

The reaction mass obtained in EXAMPLE 2, was further subjected to downstream treatment similar to that explained in EXAMPLE 1 and 100 g aqueous layer obtained post extraction with EDC was acidified with about 4.1 g HCI (30 % by weight) and heated on a oil bath maintained at about 135 °C for about 5 h. During this period methanol was continuously distilled out and simultaneous water was added drop wise to maintain reaction volume. After 5 h, the reaction mass was analyzed by HPLC and showed presence of about 4 % glucose and no presence of methyl glucoside. This corresponded to about 88 % yield of the theoretical value.

### EXAMPLE 12 - Reference

In a similar manner 551 g aqueous layer obtained post EDC extraction from EXAMPLE 2, containing 32 g of methyl glucoside was mixed with about 11.3 g of H₂SO₄, and about 0.8 g of sodium chloride. The mixture was heated in an oil bath maintained at about 135 °C for about 12 h. During this period methanol was continuously distilled out and simultaneous fresh water was added drop wise to maintain reaction volume. After 12 h, the reaction mass was analyzed by HPLC and showed presence of about 5.24 % glucose and without any methyl glucoside.

### EXAMPLE 13 - Reference

About 9.5 g methyl levulinate obtained from one of the above mentioned examples was mixed with 20 g about water and about 0.2 g H₂SO₄ and the mixture was refluxed with continuous removal of methanol 4 h. After 4 h, the reaction mass, on analysis by GC technique, was found to contain about 39 % of levulinic acid, which corresponded to about 99 % yield of theoretical value.

### EXAMPLE 14 - Reference

Molasses of about 1000 g was mixed with about 6200 g of methanol and about 170 g of sulphuric acid. The mixture was heated under pressure at about 105 °C for 5 h under stirring. After 5 h the reaction mass was cooled and centrifuged to effect solid-liquid separation. The sludge obtained after centrifugation was stirred in about 200 g of methanol followed by second solid-liquid separation. Supernatants of both solid-liquid separations were mixed together and further 17 g sulphuric acid was added to the mixture, and it was again heated under pressure at about 105 °C for about 12 h. The reaction mass on analysis by GC showed formation of about 1.7 % methyl levulinate. This reaction mass was further downstream processed in a manner stated above and the aqueous layer after solvent extraction was collected. This aqueous layer on hydrolysis resulted in a glucose solution containing about 5 % glucose by weight.

While the invention has been particularly shown and described with reference to embodiments listed in examples, it will be appreciated that several of the above disclosed and other features and functions, or alternatives thereof, may be desirably combined into many other different systems or applications.

## Claims

1. A process for the preparation of levulinic acid comprising:
(a) providing a feedstock comprising fructose and glucose;
(b) mixing methanol and an acid with said feedstock and allowing insoluble matter to separate forming a first stream;
(c) subjecting said first stream to solid-liquid separation forming a reaction mixture free of said insoluble matter;
(d) maintaining said reaction mixture at an effective temperature for a specific time period forming in a second stream, wherein the temperature is between 90° C and 120° C;
(e) subjecting said second stream to pH correction using an alkali forming a third stream;
(f) distilling said third stream forming a fourth stream and a forming a first distillate stream;
(g) filtering said fourth stream forming a fifth stream;
(h) extracting said fourth stream with an organic solvent to get a mixture of aqueous and organic phases;
(i) separating said mixture by liquid-liquid separation forming an organic stream and an aqueous stream;
(j) subjecting said organic stream to distillation to remove said organic solvent and get a product stream comprising methyl levulinate;
(k) recovering levulinic acid from said methyl levulinate by hydrolysis and removal of methanol; and
(l) recovering glucose from said aqueous stream by hydrolysis and removal of methanol.

2. The process of claim 1, wherein said feedstock comprises molasses, cane sugar syrup or inverted glucose.

3. The process of claim 1, wherein said acid is one of sulphuric acid, hydrochloric acid or methane sulphonic acid.

4. The process of claim 1, wherein said specific time period in step [b] is between 20 minutes and 300 minutes.

5. The process of claim 1, wherein pH of said second stream is between 6 and 8.

6. The process of claim 1, wherein said alkali is one of ammonia, sodium hydroxide or sodium bicarbonate.

7. The process of claim 1, wherein methanol is recovered and recycled.

8. The process of claim 1, wherein said organic solvent is one of ethylene dichloride or methyl isobutyl ketone.

9. The process of claim 1, wherein said organic solvent is recovered and recycled.

10. The process of claim 1, wherein said effective time-period in step [d] is up to 20 h.

11. The process of claim 1, wherein formic acid is recovered from said first distillate stream.

12. The process of claim 1, wherein glucose recovered from said aqueous stream is fermented to ethanol or wherein glucose recovered from said aqueous stream is enzymatically inverted to fructose.

13. The process of claim 1, wherein yield of levulinic acid is at least 60 % by weight of fructose.

14. The process of claim 1, wherein recovery of unreacted glucose in the form of fermentable glucose at least 90 % by weight of glucose.

## Patentansprüche

1. Verfahren zur Herstellung von Lävulinsäure, umfassend:
(a) Bereitstellen eines Ausgangsmaterials, umfassend Fruktose und Glukose;
(b) Mischen von Methanol und einer Säure mit dem genannten Ausgangsmaterial und Gestatten, dass sich unlösliches Material abtrennt, einen ersten Strom bildend;
(c) Aussetzen des genannten ersten Stroms einer Fest-Flüssig-Trennung, eine Reaktionsmischung ergebend, die frei von unlöslichem Material ist;
(d) Halten der genannten Reaktionsmischung bei einer effektiven Temperatur über einen spezifischen Zeitraum, einen zweiten Strom bildend, wobei die Temperatur zwischen 90° C and 120° C liegt;
(e) Aussetzen des genannten zweiten Stroms einer pH-Korrektur unter Verwendung eines Alkalis, einen dritten Strom bildend;
(f) Destillieren des genannten dritten Stroms, einen vierten Strom und einen ersten Destillatstrom bildend;
(g) Filtrieren des genannten vierten Stroms, einen fünften Strom bildend;
(h) Extrahieren des genannten vierten Stroms mit einem organischen Lösemittel, um eine Mischung von wässriger und organischer Phase zu erhalten;
(i) Trennen der genannten Mischung durch Flüssig-Flüssig-Trennung, einen organischen Strom und einen wässrigen Strom bildend;
(j) Aussetzen des genannten organischen Stroms einer Destillation, um das genannte organische Lösemittel zu entfernen und einen Produktstrom zu erhalten, der Methyllävulinat umfasst;
(k) Rückgewinnen von Lävulinsäure aus dem genannten Methyllävulinat mittels Hydrolyse und dem Entfernen von Methanol; und
(l) Rückgewinnen von Glukose aus dem genannten wässrigen Strom mittels Hydrolyse und dem Entfernen von Methanol.

2. Verfahren, nach Anspruch 1, wobei das genannte Ausgangsmaterial Melassen, Rohrzuckersirup oder Invertzucker umfasst.

3. Verfahren, nach Anspruch 1, wobei die genannte Säure eine ist von Schwefelsäure, Chlorwasserstoffsäure oder Methansulfonsäure.

4. Verfahren, nach Anspruch 1, wobei der genannte spezifische Zeitraum in Schritt [b] zwischen 20 Minuten und 300 Minuten liegt.

5. Verfahren, nach Anspruch 1, wobei der pH des genannten zweiten Stroms zwischen 6 und 8 ist.

6. Verfahren, nach Anspruch 1, wobei das genannte Alkali eines ist von Ammoniak, Natriumhydroxid oder Natriumbicarbonat.

7. Verfahren, nach Anspruch 1, wobei Methanol zurückgewonnen und aufbereitet wird.

8. Verfahren, nach Anspruch 1, wobei das genannte organische Lösemittel eines ist von Ethylendichlorid oder Methylisobutylketon.

9. Verfahren, nach Anspruch 1, wobei das genannte organische Lösemittel zurückgewonnen und aufbereitet wird.

10. Verfahren, nach Anspruch 1, wobei der genannte effektive Zeitraum in Schritt [d] bis zu 20 Stunden ist.

11. Verfahren, nach Anspruch 1, wobei Ameisensäure aus dem genannten ersten Strom zurückgewonnen wird.

12. Verfahren, nach Anspruch 1, wobei Glukose, die aus dem genannten wässrigen Strom zurückgewonnen wurde, zu Alkohol fermentiert wird, oder wobei Glukose, die aus dem genannten wässrigen Strom zurückgewonnen wurde, enzymatisch zu Fruktose invertiert wird.

13. Verfahren, nach Anspruch 1, wobei die Ausbeute an Lävulinsäure wenigstens 60 Gewichts-% der Fructose beträgt.

14. Verfahren, nach Anspruch 1, wobei das Zurückgewinnen nicht umgesetzter Glukose in Form von fermentierbarer Glukose wenigstens 90 Gewichts-% der Glukose beträgt.

## Revendications

1. Un procédé de préparation d'acide lévulinique comprenant :
(a) le fait de fournir une charge comprenant du fructose et du glucose ;
(b) le fait de mélanger du méthanol et un acide avec ladite charge et de laisser les matières insolubles se séparer de façon à former un premier courant ;
(c) le fait de soumettre ledit premier courant à une séparation solide-liquide formant un mélange réactionnel exempt de ladite matière insoluble ;
(d) le fait de maintenir ledit mélange réactionnel à une température efficace pendant une période de temps spécifique se formant en un deuxième courant, la température étant comprise entre 90° C et 120°C ;
(e) le fait de soumettre ledit deuxième courant à une correction de pH à l'aide d'un alcali formant un troisième courant ;
(f) le fait de distiller ledit troisième courant de façon à former un quatrième courant et à former un premier courant de distillat ;
(g) le fait de filtrer ledit quatrième courant de façon à former un cinquième courant ;
(h) le fait d'extraire ledit quatrième courant avec un solvant organique pour obtenir un mélange de phases aqueuse et organique ;
(i) le fait de séparer ledit mélange par séparation liquide-liquide de façon à former un courant organique et un courant aqueux ;
(j) le fait de soumettre ledit courant organique à une distillation pour éliminer ledit solvant organique et obtenir un courant de produit comprenant du lévulinate de méthyle ;
(k) le fait de récupérer l'acide lévulinique à partir dudit lévulinate de méthyle par hydrolyse et élimination du méthanol : et
(l) le fait de récupérer le glucose dudit courant aqueux par hydrolyse et élimination du méthanol.

2. Le procédé selon la revendication 1, dans lequel ladite charge comprend de la mélasse, du sirop de sucre de canne ou du glucose inversé.

3. Le procédé selon la revendication 1, dans lequel ledit acide est l'un parmi l'acide sulfurique, l'acide chlorhydrique et l'acide méthane sulfonique.

4. Le procédé selon la revendication 1, dans lequel ladite période de temps spécifique à l'étape [b] est comprise entre 20 minutes et 300 minutes.

5. Le procédé selon la revendication 1, dans lequel le pH dudit deuxième courant est compris entre 6 et 8.

6. Le procédé selon la revendication 1, dans lequel ledit alcali est l'un parmi l'ammoniaque, l'hydroxyde de sodium et le bicarbonate de sodium.

7. Le procédé selon la revendication 1, dans lequel le méthanol est récupéré et recyclé.

8. Le procédé selon la revendication 1, dans lequel ledit solvant organique est l'un parmi le dichlorure d'éthylène et la méthylisobutylcétone.

9. Le procédé selon la revendication 1, dans lequel ledit solvant organique est récupéré et recyclé.

10. Le procédé selon la revendication 1, dans lequel ladite période de temps effective à l'étape [d] va jusqu'à 20 h.

11. Le procédé selon la revendication 1, dans lequel l'acide formique est récupéré dudit premier courant de distillat.

12. Le procédé selon la revendication 1, dans lequel le glucose récupéré dudit courant aqueux est fermenté en éthanol ou dans lequel le glucose récupéré dudit courant aqueux est inversé enzymatiquement en fructose.

13. Le procédé selon la revendication 1, dans lequel le rendement en acide lévulinique est d'au moins 60% en poids de fructose.

14. Le procédé selon la revendication 1, dans lequel la récupération du glucose n'ayant pas réagi sous forme de glucose fermentescible à au moins 90% en poids de glucose.
